(19) Europäisches Patentamt
European Patent Office
Office européen des brevets

(11) Veröffentlichungsnummer : **0 334 083 B1**

(12) # EUROPÄISCHE PATENTSCHRIFT

(45) Veröffentlichungstag der Patentschrift : **24.07.91 Patentblatt 91/30**

(51) Int. Cl.[5] : **A61K 31/675**, A61K 47/00

(21) Anmeldenummer : **89103844.0**

(22) Anmeldetag : **04.03.89**

(54) **Ifosfamid-Mesna-Lyophilisat und Verfahren zu dessen Herstellung.**

(30) Priorität : **19.03.88 DE 3809337**

(43) Veröffentlichungstag der Anmeldung : **27.09.89 Patentblatt 89/39**

(45) Bekanntmachung des Hinweises auf die Patenterteilung : **24.07.91 Patentblatt 91/30**

(84) Benannte Vertragsstaaten : **AT BE CH DE ES FR GB GR IT LI LU NL SE**

(56) Entgegenhaltungen :
**EP-A- 0 002 495**
**EP-A- 0 251 657**
**EP-A- 0 265 812**

(73) Patentinhaber : **ASTA Pharma Aktiengesellschaft**
**Weismüllerstrasse 45**
**W-6000 Frankfurt am Main 1 (DE)**

(72) Erfinder : **Sauerbier, Dieter**
**Rauhe Horst 18**
**W-4806 Werther (DE)**
Erfinder : **Isaac, Otto, Dr.**
**Liesingstrasse 8**
**W-6450 Hanau 9 (DE)**
Erfinder : **Brade, Wolfgang Peter, Dr.**
**Im Banngarten 2**
**W-6393 Wehrheim 4 (DE)**

**EP 0 334 083 B1**

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue Saint-Denis, 75001 PARIS

**Beschreibung**

Der chemische Name für den Wirkstoff Ifosfamid ist 3-(2-Chlorethyl)-2-(chlorethylamino)-tetrahydro-2H-1,3,2-oxazaphosphorin-2-oxid

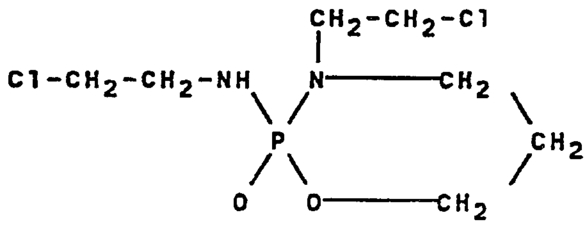

Ifosfamid gehört wie Cyclophosphamid zur chemischen Gruppe der Oxazaphosphorine und wird therapeutisch zur Behandlung von Tumor-Erkrankungen eingesetzt.

Der chemische Name für den Uroprotektor Mesna ist Natrium-2-mercaptoethansulfonat

$$Na^+\{HS-CH_2-CH_2-SO_3\}^-$$

Mesna schützt beispielsweise die harnableitenden Organe bei der Therapie von Tumor-Erkrankungen mit Ifosfamid, wobei diese uroprotektive Wirkung von Mesna insbesondere bei gleichzeitiger und synchroner Verabreichung zusammen mit dem Ifosfamid gegeben ist. Diese Technik ist z.B. in EP-A-0 002 495 beschrieben.

Ifosfamid ist ein weißes, kristallines Pulver mit einem Schmelzpunkt von 48°C bis 51°C und stark hygroskopischen Eigenschaften. Bereits unterhalb des Schmelzpunktes beginnt Ifosfamid zu sintern ; es muß deshalb bei möglichst niedrigen Temperaturen (Raumtemperatur und darunter) gelagert werden. Außerdem ist ein Kontakt mit Luftfeuchigkeit möglichst zu vermeiden.

Ifosfamid löst sich zu etwa 10 Gewichtsprozent in Wasser, ist aber in wässriger Lösung nur begrenzt haltbar (maximal 3 bis 4 Stunden bei 20°C bis 22°C beziehungsweise 36 Stunden bei 4 bis 6°C).

Ifosfamid wird ausschließlich parenteral appliziert. Die Injektionsflaschen enthalten 200 bis 5000 mg Ifosfamid in Form eines Sterilkristallisats, das vor der Applikation in Wasser für Injektionszwecke gelöst wird, so daß eine 4%ige Konzentration nicht überschritten wird. Diese Lösung ist zur intravenösen Injektion geeignet. Zur intravenösen Kurzinfusion wird die Ifosfamid-Lösung in 500 ml Ringer-Lösung oder ähnlichen Infusionsflüssigkeiten aufgelöst. Die Infusionsdauer beträgt ca. 30 Minuten, eventuell 1 bis 2 Stunden. Bei der 24-Stunden-Infusion wird die Ifosfamid-Lösung beispielsweise in insgesamt 3 Liter 5% Dextrose-Kochsalzlösung aufgelöst.

Ifosfamid verursacht bei der Herstellung und Verarbeitung mannigfaltige Probleme. Bei der Herstellung des steril kristallisierten Ifosfamids resultiert ein Produkt von wechselnder physikalischer Beschaffenheit. Durch die unterschiedliche Rieselfähigkeit wird insbesondere die Dosierungsgenauigkeit bei der Abfullung in hohem Maße beeinträchtigt.

Die Verarbeitung des Ifosfamids wird weiterhin erschwert durch seine Hygroskopizität und den niedrigen Schmelzpunkt. Bei längerer Lagerung sintert das Sterilkristallisat und die Lösungsgeschwindigkeit vermindert sich. Mit beginnender Sinterung des Ifosfamids nehmen auch die Klarlöslichkeit und der pH-Wert der Lösung bei gleichzeitiger Gelbfärbung ab ; eine therapeutische Verwendung ist dann im allgemeinen nicht mehr möglich. Ein Zytostatikum-lyophilisat enthaltend Galactitol ist aus EP-A-0 265 812 bekannt und weist eine verbesserte Stabilität auf.

Mesna ist ebenfalls eine Substanz, die nur unter besonderen Bedingungen stabil und haltbar ist.

Eine Kombinationsmöglichkeit aus Ifosfamid und Mesna, welches einen großen Vorteil hinsichtlich Lagerung und praktischer Handhabung darstelllen würde, existiert bis jetzt nicht.

Aufgabe der Erfindung ist es daher, Ifosfamid und Mesna in einer Form mit verbesserten Eigenschaften, wie verbesserte pharmazeutische Qualität, Dosierbarkeit und Löslichkeit, bereitzustellen, die leichter anzuwenden ist, und insbesondere zur Herstellung von injizierbaren Lösungen geeignet ist.

Es wurde nun Überraschend gefunden, daß die bisherigen Nachteile und Schwierigkeiten bei der Handhabung und Lagerung von Ifosfamid und Mesna durch Verwendung eines bestimmten Ifosfamid-Mesna-Lyophilisats behoben werden können. Insbesondere ist es überraschend, daß das erfindungsgemäße Lyophilisat eine größere Thermostabilität des Ifosfamids besitzt als die bislang verwendete Ifosfamid-Trockenabfüllung.

Trockenabfüllungen mit Ifosfamid sind bei 40°C bereits nach einer Lagerzeit von 1 Monat nachgedunkelt; nach 2 Monaten ist der Flascheninhalt gesintert und gelb verfärbt. Bei einer Lagerungstemperatur von 55°C ist das trocken abgefüllte Ifosfamid bereits innerhalb von 4 Tagen geschmolzen.

Demgegenüber ist bei erfindungsgemäß hergestellten Lyophilisat unter den vorgenannten Lagerbedingungen weder eine Verfärbung noch eine Veränderung der Konsistenz des Ifosfamids erkennbar. Ebenfalls zeigen sich keine Veränderungen bei dem Mesna.

Die Lösungsgeschwindigkeit des Ifosfamid-Mesna-Lyophilisats ist gegenüber der Ifosfamid-Trocken-abfüllung deutlich erhöht. Während sich das Lyophilisat unabhängig von der Lagerdauer bei der Zugabe des Lösungsmittels sofort löst, müssen die Injektionsflaschen mit der Trockenabfüllung nach Einspritzen des Lösungsmittels 1/2 bis 3 Minuten kräftig geschüttelt werden. Wenn hierbei die Auflösung nicht sofort restlos erfolgt, und dies ist bei länger gelagerten Injektionsflaschen der Fall, ist es sogar erforderlich, die Lösung einige Minuten stehen zu lassen. Die Anwendung des Präparates in der Klinik wird dadurch erschwert.

Ifosfamid-Mesna-Lyophilisat zeigt im Gegensatz zu Sterilkristallisat auch nach der Lagerung von mehreren Jahren noch optimale Lösungseigenschaften.

Außerdem ist die Ifosfamid-Trockenabfüllung (das heißt das reine Ifosfamid-Kristallisat) viel empfindlicher gegen Luftfeuchtigkeit als das Lyophilisat. So verflüssigt sich die Ifosfamid-Trockenabfüllung bereits bei einer relativen Luftfeuchtigkeit von unter 75%, während das Lyophilisat selbst bei 100% relativer Luftfeuchigkeit zwar feucht wird, aber seine äußere Form behält.

Bei der Abfüllung des Sterilkristallisats ist ferner die Gefahr einer partikulären oder mikrobiellen Kontamination in wesentlich stärkerem Maße als beim Lyophilisat gegeben.

Bei der Herstellung des Ifosfamid-Mesna-Lyophilisats erfolgt die Sterilfiltration der Lösung hingegen erst unmittelbar vor der Abfüllung in die Injektionsflaschen. Dadurch ist gegenüber der Abfüllung von Sterilkristallisat eine größere mikrobiologische Sicherheit gegeben. Auch partikuläre Verunreinigungen, die bei der Trockenabfüllung gelegentlich Anlaß zu Beanstandungen geben, lassen sich durch die Filtration der Lösung mit größerer Sicherheit vermeiden.

Die Lyophilisation des Ifosfamids in Kombination mit Mesna führt jedoch nicht nur zu einer Produktverbesserung, sondern ist in der Herstellung und praktischen Anwendung auch wirtschaftlicher als die getrennte Herstellung von Sterilkristallisat und Mesna-Injektionslösung.

Darüberhinaus besitzt die erfindungsgemäße Kombination auch bei der Anwendung eine überraschende bessere Wirkung als die bisherige getrennte Applikation von Ifosfamid und Mesna :

So erfolgt beispielsweise bei der erfindungsgemäßen Kombination bei intravenöser, kontinuierlicher Infusion (zum Beispiel in der Zusammensetzung 5,0 g Ifosfamid + 2,0 g Mesna) eine kontinuierliche Uroprotektion, und zwar durch die gleichzeitige kontinuierliche renale Elimination von urotoxischen Metaboliten und Mesna. Dadurch wird der uroprotektionsmindernde Effekt einer Blasenentleerung minimiert. Die fixe Dosisrelation von Ifosfamid und Mesna im Lyophilisat vermeidet bei dem Einsatz als kontinuierliche intravenöse Infusion (zum Beispiel 5 g Ifosfamid + 2 g Mesna über 6 Stunden oder 10 g Ifosfamid + 4 g Mesna über 24 Stunden kontinuierlich infundiert) eine unzureichende Uroprotektion, wie sie durch wiederholte Bolusinjektionen von Mesna oder auch eine zu niedrige Infusionsdosis auftreten kann. Der Einsatz des Kombinationslyophilisats als Kurzzeitinfusion über 30 Minuten bis zu 2 Stunden in den Mengen von beispielsweise 500 mg bis 5 g Ifosfamid zusammen mit 20% der Ifosfamid-Menge als Mesna garantiert eine ausreichende Uroprotektion in den ersten 4 Stunden. Bevorzugte Dosierungen für die Anwendung am Menschen sind zum Beispiel :

0,5 g Ifosfamid + 0,1 g Mesna
1 g Ifosfamid + 0,2 g Mesna
2 g Ifosfamid + 0,4 g Mesna
5 g Ifosfamid + 1,0 g Mesna
5 g Ifosfamid + 2,0 g Mesna

Es hat sich gezeigt, daß nur das erfindungsgemäße Verfahren unter Verwendung eines Hexits, wie zum Beispiel Mannit, ein verbessertes Ifosfamid-Mesna-Lyophilisat ergibt. Beispielsweise konnte durch Beimischung von Kochsalz, wie sie bei Trockenabfüllungen von anderen Oxazaphosphorinen üblich ist, kein Lyophilisat erhalten werden.

Erfindungsgemäß wird beispielsweise eine wässrige Lösung, die 1-13 Gewichtsprozent an Ifosfamid und 0,05-13 Gewichtsteile Mesna enthält, sowie als Gerüstbildner 0,1-17 Gewichtsteile Hexit, bezogen auf einen Gewichtsteil Ifosfamid, gefriergetrocknet. Vorzugsweise enthält diese wässrige Lösung 5-12 Gewichtsprozent

Ifosfamid und 0,5-12 Gewichtsprozent Mesna, insbesondere 8-10 Gewichtsprozent Ifosfamid und 0,8-10 Gewichtsprozent Mesna.

Es können auch entsprechende Ethanol-Wasser-Lösungen von Ifosfamid und Mesna anstelle einer reinen wässrigen Lösung verwendet werden (Ethanolanteil einer solchen Lösung bis zu 45 m/m*), beispielsweise 1-20% Ethanol). In solchen Fällen wird möglichst zuerst das Ethanol vorzeitig im Vacuum entfernt, bevor das restliche Eis sublimiert wird. Die Bedingungen für die zuerst erfolgte Ethanolentfernung sind zum Beispiel : Druck 5-$10^{-1}$ mbar, Temperatur von −25°C auf −5°C steigend innerhalb von 10 Stunden, anschließend wird die Temperatur der Stellplatten auf 15°C erhöht. Im einzelnen hängen diese Bedingungen auch von den unterschiedlichen Schichthöhen des zu trocknenden Gutes in den Injektionsflaschen ab und sind entsprechend zu variieren.

Die Menge an Hexit in dieser wässrigen beziehungsweise wässrig-ethanolischen Lösung beträgt im allgemeinen 1-17, vorzugsweise 3-12, insbesondere 5-9 Gewichtsprozent. Bezieht man die Hexit-Menge auf einen Gewichtsteil Ifosfamid, dann ist die Hexit-Menge 0,1-17, vorzugsweise 1 bis 2,5 insbesondere 0,6-0,8 Gewichtsteile Hexit pro 1 Gewichtsteil Ifosfamid. Bezogen auf 1 Gewichtsteil Mesna beträgt die Hexit-Menge zum Beispiel 0,1-17, vorzugsweise 1-6, insbesondere 3-4 Gewichtsteile.

*) Definition gemäß Deutsches Arzneibuch 9. Ausgabe : Prozent Masse in Masse

Als Hexit kommen in Frage : Mannit, Glucit (Sorbit, wie D-Sorbit), Dulcit, Allit, Altrit (z.B. D- und L-Altrit), Idit (z.B. D- und L-Idit), deren optisch aktive Formen (D- bzw. L-Formen), sowie die entsprechenden Racemate. Insbesondere wird Mannit, wie D-Mannit, L-Mannit, DL-Mannit verwendet und zwar hiervon vorzugsweise D-Mannit. Als Hexit können auch Mischungen der genannten Hexite verwendet werden, z.B. Mischungen von Mannit und Sorbit und/oder Dulcit.

Neben dem Hexit können auch noch andere, übliche pharmazeutische Hilfsstoffe zugefügt werden, wie zum Beispiel Glycin, Lactose, Polyvinylpyrrolidon, Glukose, Fructose, Albumin und äquivalente gerüstbildende Stoffe. Die Gesamtmenge an solchen Stoffen in der Lösung, die für die Gefriertrocknung eingesetzt wird, ist beispielsweise 0-16,8 Gewichtsteile, bezogen auf 1 Gewichtsteil Ifosfamid bzw. Mesna. In dem fertigen Lyophilisat kann die Gesamtmenge an solchen Hilfsstoffen bis zu 16,8 Gewichtsteile, bezogen auf einen Gewichtsteil Hexit, betragen. Im einzelnen richtet sich die Menge an solchen Hilfsstoffen nach der vorhandenen Menge Hexit und zwar derart, daß die Gesamtmenge an Hexit und solchen anderen Hilfsstoffen in dem fertigen Lyophilisat maximal nicht mehr als 17 Gewichtsteile beträgt, bezogen auf 1 Teil Ifosfamid bzw. Mesna. Falls in dem Lyophilisat beispielsweise nur 0,1 Gewichtsteile Hexit vorliegen, können also bis zu 16,9 Gewichtsteile an anderen Hilfsstoffen vorliegen ; falls beispielsweise 8,5 Gewichtsteile Hexit vorliegen, kann z.B. die Menge an anderen Hilfsstoffen bis zu 8,5 Gewichtsteile, bezogen auf 1 Teil Ifosfamid bzw. Mesna, betragen.

Zur Herstellung der für die Gefriertrocknung einzusetzenden Lösung werden etwa 70 bis 80%,vorzugsweise 75% der erforderlichen Wassermenge bzw. ethanolischer Wassermenge vorgelegt und die entsprechenden Mengen Ifosfamid, Mesna und Mannit nacheinander (das heißt erst wird das Ifosfamid, dann das Mesna und anschließend der Hexit (z.B. Mannit) unter ständigem Rühren beziehungsweise unter ständiger Bewegung gelöst. Das zur Herstellung der Lösung verwendete Wasser wird zwecks Verdrängung von Sauerstoff mit einem inerten Gas wie zum Beispiel Stickstoff, Kohlendioxid oder einem Edelgas begast. Auch während der Herstellung der Lösung wird das inerte Gas in die Lösung eingeleitet. Die Verdrängung von Sauerstoff ist wichtig, da Mesna leicht zum Disulfid oxydiert wird. Nach vollständiger Auflösung wird auf das Endvolumen aufgefüllt und der pH-Wert gemessen. Der pH-Wert dieser Lösung soll beispielsweise nach dem Verdünnen zwischen 4 und 7 liegen. Vorzugsweise wird zur pH-Messung eine 4%ige Lösung, bezogen auf Ifosfamid, hergestellt.

Die so erhaltene Ifosfamid-Mesna-Lösung wird dann durch Filtration über hierfür übliche, keimdichte Filter sterilisiert, als Druckgas wird Stickstoff verwendet. Die Aufbewahrungszeit bis zur Abfüllung in die Injektionsbehälter soll einschließlich der Zeit der Lösungsherstellung eine Zeit von 3-4 Stunden nicht überschreiten, sofern es sich um Raumtemperatur (18°C bis 22°C) handelt.

Falls die anschließende Gefriertrocknung noch nicht sofort möglich ist, kann eine solche Lösung, gegebenenfalls auch nach Abfüllung in die Injektionsbehälter, beispielsweise noch bis zu 36 Stunden bei niedrigen Temperaturen, beispielsweise zwischen −5° und +10°C, vorzugsweise +4° bis +6°C, aufbewahrt werden, bevor die Gefriertrocknung beginnt.

Zur Durchführung des erfindungsgemäßen Verfahrens wird dann die so erhaltene Ifosfamid-Mesna-Lösung in Behälter für Injektionspräparate, beispielsweise Ampullen oder andere Glasgefäße eingefüllt. Die Behälter werden vor und nach der Befüllung mit sterilen und partikelfreien inerten Gas (z.B. Stickstoff) begast. Anschließend werden die Gefriertrocknungsstopfen aufgesetzt und lyophilisiert.

Zur Sterilisation werden übliche keimdichte Filter, beispielsweise übliche Bakterienfilter mit einer Porengröße von etwa 0,2 µm verwendet. Die verwendeten Glasgefäße beziehungsweise Ampullen werden vorher in üblicher Weise sterilisiert.

Der verwendete Hexit (vorzugsweise Mannit, insbesondere D-Mannit) soll den Anforderungen der Britischen Pharmakopoeia 1980 entsprechen.

Der eingesetzte Hexit muß pyrogenfrei sein (Pyrogene sind Fieber erzeugende Endotoxine, die von Bakterien gebildet werden). Dasselbe gilt für das verwendete Ifosfamid und Mesna. Die Entfernung bzw. Zerstörung der Pyrogene erfolgt auf übliche Weise (beispielsweise wird die Wirkstofflösung vor der Sterilfiltration mit Aktivkohle behandelt). Ebenfalls muß das verwendete Injektionswasser steril und pyrogenfrei sein und den Anforderungen des Deutschen Arzneibuches, 9. Ausgabe 1986 entsprechen.

Als Injektionsgefäße werden zweckmäßig solche aus Röhrenglas beziehungsweise Hüttenglas der III hydrolytischen Klasse verwendet (beispielsweise 10 R, 30 R und 50 H). (Siehe hierzu Deutsches Arzneibuch, 9. Ausgabe 1986 Seiten 161-164 und DIN-Normen 58366, Teil 1 und Teil 5). Weiterhin sollen die Injektionsgefäße sowie die weiteren Hilfsstoffe, wie Gummistopfen und Bördelkappen, den Anforderungen der DIN-Normen 58366, Teil 2 und Teil 3 sowie 58367, Teil 1 entsprechen.

Die Lösungsmengen der Ifosfamid-Mesna-Lösungen, die für die Lyophilisation vorgesehen sind, in den jeweiligen Behältern (Ampullen) oder sonstigen Behältern für Injektionspräparate liegen pro Behälter zum Beispiel zwischen 1 und 500, vorzugsweise 1 und 250, insbesondere 2 und 50 ml. Die Behälter sind jeweils so zu bemessen, daß das hierin enthaltene Lyophilisat später in einer größeren Menge Flüssigkeit aufgelöst werden kann. Sie sollen daher im allgemeinen ein Volumen besitzen, das ausreicht, um eine gebrauchsfertige Endlösung herzustellen, die etwa das 2 bis 5, vorzugsweise 2 bis 4, insbesondere 2 bis 2,5 fache des Volumens der ursprünglich eingefüllten Lyophilisat-Lösung hat.

Wie bereits erwähnt, wird vorzugsweise jede Ampulle beziehungsweise jedes Glasgefäß mit einer Einzeldosis von Ifosfamid und Mesna gefüllt, wobei die Ifosfamid-Menge pro Glasgefäß beispielsweise zwischen 100 mg bis 10 g, vorzugsweise 200 mg bis 5 g, die Mesna-Menge 10 mg bis 10 g, vorzugsweise 20 mg bis 5 g beträgt. Anschließend wird die Lösung in diesem Glasgefäß oder der Ampulle in herkömmlicher Weise gefriergetrocknet. Es ist jedoch auch möglich, größere Mengen Ifosfamid-Mesna, das heißt ein entsprechend größeres Lösungsvolumen der Ifosfamid-Mesna-Lösung in eimem entsprechend größeren Gefäß zu lyophilisieren, und anschließend das erhaltene Lyophilisat in entsprechende kleinere Dosierungen zu unterteilen beziehungsweise abzupacken.

Die Lyophilisierung selbst wird so durchgeführt, daß die Ampullen oder Glasgefäße oder sonstige Gefäße, welche die Ifosfamid-Mesna-Lösung enthalten, unmittelbar auf eine Stellplatte oder in Tabletts auf einer Stellplatte in eine Gefriertrocknungskammer eingestellt werden. Nach dem Verschließen der Kammer werden die Ampullen beziehungsweise Gefäße auf Temperaturen unter 0°C abgekühlt, sodaß das Wasser vollständig ausfriert. Beispielsweise wird auf Temperaturen zwischen −70°C bis 0°C vorzugsweise zwischen −70°C und −5°C, insbesondere −50°C bis −30°C, oder −45°C bis −35°C abgekühlt. Sobald die Lösungen vollständig gefroren sind, wird die Gefriertrocknungs-kammer allmählich evakuiert und mit dem Trocknen begonnen. Hierbei wird zuerst das nicht-adsorptiv gebundene Lösungsmittel entfernt und zwar bei Temperaturen zwischen −30°C bis +40°C, vorzugsweise 0°C bis +30° C, insbesondere +10°C bis +20°C, wobei ein Druck zwischen $10^{-3}$ bis 6, vorzugsweise $10^{-2}$ bis 2, insbesondere $10^{-1}$ bis 1 mbar eingestellt wird. Bei den zuvor angegebenen Temperaturen beziehungsweise Tempe-raturbereichen handelt es sich um die Temperatur der Stellplatten. Der Prozeß wir dabei so gesteuert, daß die über die Plattentemperatur zugeführte Wärme vollständig als Sublimationswärme verbraucht wird, und die Temperatur der gefrorenen Ifosfamid-Mesna-haltigen Lösung stets unterhalb ihrer eutektischen Temperatur bleibt. Die jeweils gewünschte Temperatur der Stellplatte kann zum Beispiel durch Programmscheiben oder Computer programmiert werden. Die Dauer zur Entfernung dieses nichtadsorptiv gebundenen Lösungsmittels ist von der Größe der einzelnen Behälter abhängig und liegt beispielsweise zwischen etwa 8 bis 50 Stunden bei einer Plattentemperatur von +15°C und einem Druck von 0,8 mbar. Beispielsweise wird in diesem Zusammenhang auf die in dem Beispiel angegebenen Zeiten verwiesen.

Die vollständige Entfernung des nicht-adsorptiv gebundenen Wassers zeigt sich wie folgt an : Nicht adsorptiv gebundenes Wasser liegt als Eis vor. Durch die sogenannte Druckanstiegsmessung wird festgestellt, ob derartiges Wasser noch im Lyophilisat vorhanden ist. Dazu wird ein Ventil zwischen Trockenkammer und Kondensatorraum, an dem auch die Vakuumpumpe angeschlossen ist, geschlossen. Vorhandenes Eis würde dann schnell sublimieren und einen Druckanstieg in der Trockenkammer herbeiführen. Bei der Druckanstiegmessung darf der Druck in der Kammer nach 15 Minuten vom Ausgangswert, zum Beispiel 0,8 mbar, höchstens auf 1 mbar ansteigen. Ein stärkerer Anstieg würde bedeuten, daß die Haupttrocknung noch nicht abgeschlossen ist.

Das noch vorhandene, restliche adsorptiv gebundene Lösungsmittel wird dann durch eine Nachtrocknung entfernt. Diese beträgt beispielsweise 3 bis 12 Stunden bei einem Vakuum von $10^{-1}$ bis $10^{-4}$ mbar, insbesondere 3-4 Stunden bei einem Vakuum von $10^{-3}$ bis $10^{-4}$ mbar.

Der Lyophilisationsprozeß ist beendet, wenn die Restfeuchte (bestimmt nach K. Fischer) unter 1%, vorzugsweise unter 0,5 liegt. Insbesondere erfolgt die Nachtrocknung zur Entfernung von adsorptiv gebundenem Wasser bei Temperaturen zwischen 0 bis 40, vorzugsweise 10 bis 35, insbesondere 20 bis 30°C und einem

Druck zwischen $10^{-4}$ bis $10^{-1}$, vorzugsweise $10^{-3}$ bis $10^{-2}$, insbesondere $10^{-3}$ bis $5 \times 10^{-3}$ mbar, wobei diese Nachtrocknung beispielsweise 2 bis 36, vorzugsweise 6 bis 24, insbesondere 3 bis 12 Stunden in Anspruch nimmt.

Nach Beendigung der Gefriertocknung werden die Gefäße verschlossen. Das erfindungsgemäße Verfahren wird in sämtlichen Stufen unter aseptischen Bedingungen durchgeführt.

Der Verschluß der Injektionflaschen erfolgt dann zum Beispiel nach Belüftung der Gefriertrocknungskammer auf Normaldruck durch Zufuhr eines trockenen inerten Gases (z.B. Stickstoff) mit besonderen Gefrietrocknungs-Gummistopfen, die zur Vermeidung von Abrieb und Zwecks Verbesserung der Gleitfähigkeit silikonisiert sind.

Mit Ausnahme des Einfrierens und der Entfernung des Lösungsmittels im Vakuum erfolgen alle Operationen unter inerter Gasatmosphäre (z.B. Stickstoff, Kohlendioxid, Edelgase).

Beispiel 1

Zur Gefriertrocknung wird folgende Lösung eingesetzt :

| | | |
|---|---|---|
| Mesna | | 20 mg |
| Ifosfamid | | 100 mg |
| D-Mannit | | 70 mg |
| Injektionswasser | ad | 1 ml |

Die Dichte dieser Lösung beträgt 1,061 g/ml bei +20°C.

Die anzusetzende Lösungsmenge richtet sich nach der jeweiligen Abfüll- und Gefriertrocknungs-Kapazität.

Sämtliche Verfahrensschritte bei der Herstellung der Lösung und der Abfüllung werden unter Stickstoff beziehungsweise Stickstoffbegasung durchgeführt.

Herstellung der Lösung :

Es werden ca. 80% Injektionswassermenge vorgelegt und die entsprechenden Menge Mesna, Ifosfamid und Mannit in dem Wasser nacheinander unter ständigem Rühren und Stickstoffbegasung gelöst. Nach vollständiger Auflösung wird auf das Endvolumen aufgefüllt und der pH-Wert gemessen.

Die fertige Lösung wird durch Filtration über hierfür übliche keimdichte Filter sterilisiert (zum Beispiel Sartorius SM 11107 oder SM 11307, 0,2 µm Porenweite, Pall Filter NRP (Porenweite 0,2 µm) und unter Vermeidung partikulärer und bakterieller Kontamination bis zur Abfüllung aufbewahrt. Als Druckgas bei der Filtration wird Stickstoff verwendet. Eine Lagerung bei Raumtemperatur (20-22°C) soll einschließlich der Zeit der Lösungsherstellung 3-4 Stunden nicht überschreiten. Bei nicht sofortiger anschließender Gefriertrocknung kann die Lösung noch etwa 36 Stunden bei +4°C bis +6°C aufbewahrt werden.

Zur Sterilfiltration können zusätzlich übliche Vorfilter (zum Beispiel Sartorius SM 13400 oder Pall LPA) zum Schutz der Sterilfilter eingesetzt werden.

Reinigung der Injektionsflaschen :

Die Injektionsflaschen werden mit demineralisiertem Wasser warm und kalt und Luft gespült. Sämtliche Reinigungsmedien werden durch Filtration von Schwebstoffen befreit.

Unter Vermeidung von Rekontamination durch Partikel aus der Luft werden die Flaschen mittels Heißluft getrocknet und sterilisiert (diskontinuierlich bei 180°C/2 Stunden).

Die Reinigung der Gummistopfen, mit denen die Injektionsflaschen verschlossen werden, erfolgt unter Verwendung von demineralisiertem Wasser und beispielsweise einem Reinigungsmittel, bestehend aus nichtionogenen Tensiden und Phosphorsäureestern in wässriger Lösung.

Die gereinigten Stopfen werden unter Verwendung von demineralisiertem Wasser oder filtriertem demineralisiertem Wasser faser- und flusenfrei gespült. Die so gereinigten Stopfen werden dann mittels Dampf sterilisiert.

Die so gereinigten und sterilisierten Injektionsflaschen werden nun aseptisch mit der Ifosfamid-Mesna-Lösung gefüllt und mit dem Gummistopfen versehen, wobei die Behälter vor und nach der Füllung mit Stickstoff begast werden.

| Ifosfamid | Mesna | Füllmenge | Anwendungsvolumen* |
|---|---|---|---|
| 200 mg | 40 mg | 2 ml | 5 ml |
| 500 mg | 100 mg | 5 ml | 12,5 ml |
| 1 g | 0,2 g | 10 ml | 25 ml |
| 2 g | 0,4 g | 20 ml | 50 ml |
| 5 g | 1,0 g | 50 ml | 125 ml |
| 5 g | 2,0 g | 50 ml | 125 ml |

* für die spätere Verdünnung des Lyophilisats

Die Füllvolumina sollen folgende Grenzen nicht überschreiten :

| Füllvolumen | Grenzwerte der Einzelfüllvolumina | Durchschnittsgrenzwerte des Füllvolumens |
|---|---|---|
| 2 ml | 1,9 - 2,1 ml | 1,95 - 2,05 ml |
| 5 ml | 4,8 - 5,2 ml | 4,9 - 5,1 ml |
| 10 ml | 9,7 - 10,3 ml | 9,85 - 10,15 ml |
| 20 ml | 19,4 - 20,6 ml | 19,7 - 20,3 ml |
| 50 ml | 48,5 - 51,5 ml | 49,25 - 50,75 ml |

Die Füllvolumina sind statistisch zu überwachen, wobei mindestens alle 30 Minuten das Füllvolumen je Füllstelle einmal gemessen werden soll.

Die abgefüllten Injektionsflaschen werden so schnell wie möglich auf −40°C eingefroren.

Die Bedingungen für die Gefriertrocknung sind für die einzelnen Größen der Injektionsflaschen unterschiedlich. Es gelten beispielsweise die folgenden Werte :

Dauer der Haupttrocknung bei einer Plattentemperatur von +15°C und 0,6 mbar :

| | |
|---|---|
| ca. 8-10 Stunden | für Gefäße mit 200 mg Ifosfamid + 40 mg Mesna |
| ca. 12-15 Stunden | für Gefäße mit 500 mg Ifosfamid + 100 mg Mesna |
| ca. 13-16 Stunden | für Gefäße mit 1000 mg Ifosfamid + 200 mg Mesna |
| ca. 25-32 Stunden | für Gefäße mit 2000 mg Ifosfamid + 400 mg Mesna |
| ca. 44-50 Stunden | für Gefäße mit 5000 mg Ifosfamid + 1000 mg Mesna |

Dauer der Nachtrocknung ca. 3-4 Stunden unter Vakuum von $5 \times 10^{-4}$ mbar, bei einer Plattentemperatur von +25°C.

Die Restfeuchte (nach K. Fischer bestimmt) soll unter 0,5% liegen.

Nach Beendigung der Gefriertrocknung werden die Injektionsflaschen verschlossen.

Zur Sicherung der Gummistopfen werden Bördelkappen aufgesetzt und anrolliert. Die fertigen Injektionsflaschen werden auf mechanische Defekte (Sprünge, fehlerhafter Verschluß etc.) kontrolliert.

Beispiel 2

Zur Gefriertrocknung wird folgende Lösung eingesetzt :

| | |
|---|---|
| Mesna | 100 mg |
| Ifosfamid | 100 mg |
| D-Mannit | 70 mg |
| Inj.-Wasser ad | 1 ml |

Die Dichte dieser Lösung beträgt 1,101 g/ml bei +20°C Die anzusetzende Lösungsmenge richtet sich nach der jeweiligen Abfüll- und Gefriertrocknungs-Kapazität.

Sämtliche Verfahrensschritte bei der Herstellung der Lösung und der Abfüllung werden unter Stickstoff beziehungsweise Stickstoffbegasung durchgeführt.

Herstellung der Lösung :

Es werden ca. 80% Injektionswassermenge vorgelegt und die entsprechende Menge Mesna, Ifosfamid und Mannit in dem Wasser nacheinander unter ständigem Rühren und Stickstoffbegasung gelöst. Nach vollständiger Auflösung wird auf das Endvolumen aufgefüllt und der pH-Wert gemessen.
Die fertige Lösung wird durch Filtration über hierfür übliche keimdichte Filter sterilisiert (zum Beispiel 0,2 µm Porenweite) und unter Vermeidung partikulärer und bakterieller Kontamination bis zur Abfüllung aufbewahrt. Als Druckgas bei der Filtration wird Stickstoff verwendet. Eine Lagerung bei Raumtemperatur (20-22°C) soll einschließlich der Zeit der Lösungsherstellung 3-4 Stunden nicht überschreiten. Bei nicht sofortiger anschließender Gefriertrocknung kann die Lösung noch etwa 36 Stunden bei +4°C bis +6°C aufbewahrt werden.

Zur Sterilfiltation können zusätzlich übliche Vorfilter (zum Beispiel Sartorius SM 13400 oder Pall LPA) zum Schutz der Sterilfilter eingesetzt werden.

Reinigung der Injektionsflaschen :

Die Injektionsflaschen werden mit demineralisiertem Wasser warm und kalt und Luft gespült. Sämtliche Reinigungsmedien werden durch Filtration von Schwebstoffen befreit.
Unter Vermeidung von Rekontamination durch Partikel aus der Luft werden die Flaschen mittels Heißluft getrocknet und sterilisiert (diskontinuierlich bei 180°C/2 Stunden).
Die Reinigung der Gummistopfen, mit denen die Injektionsflaschen verschlossen werden, erfolgt unter Verwendung von demineralisiertem Wasser und beispielsweise einem Reinigungsmittel, bestehend aus nicht ionogenen Tensiden und Phosphorsäureestern in wässriger Lösung.
Die gereinigten Stopfen werden unter Verwendung von demineralisiertem Wasser oder filtriertem demineralisiertem Wasser faser-und flusenfrei gespült.

Die so gereinigten Stopfen werden dann mittels Dampf sterilisiert.
Die so gereinigten und sterilisierten Injektionsflaschen werden nun aseptisch mit der Ifosfamid-Mesna-Lösung gefüllt und mit dem Gummmistopfen versehen, wobei die Behälter vor und nach der Füllung mit Stickstoff begast werden.

Füllmengen :

| Ifosfamid | Mesna | Füllmenge | Anwendungsvolumen* |
|---|---|---|---|
| 200 mg | 200 mg | 2 ml | 5 ml |
| 500 mg | 500 mg | 5 ml | 12,5 ml |
| 1 g | 1 g | 10 ml | 25 ml |
| 2 g | 2 g | 20 ml | 50 ml |
| 5 g | 5 g | 50 ml | 125 ml |

* für die spätere Verdünnung des Lyophilisates *

Die Füllvolumina sollen folgende Grenzen nicht überschreiten :

| Füllvolumen | Grenzwerte der Einzelfüllvolumina | Durchschnittswerte des Füllvolumen |
|---|---|---|
| 2 ml | 1,9 - 2,1 ml | 1,95 - 2,05 ml |
| 5 ml | 4,8 - 5,2 ml | 4,9 - 5,1 ml |
| 10 ml | 9,7 - 10,3 ml | 9,85 - 10,15 ml |
| 20 ml | 19,4 - 20,6 ml | 19,7 - 20,3 ml |
| 50 ml | 48,5 - 51,5 ml | 49,25 - 50,75 ml |

Die Füllvolumina sind statistisch zu überwachen, wobei mindestens alle 30 Minuten das Füllvolumen je Füllstelle einmal gemessen werden soll.

Die abgefüllten Injektionsflaschen werden so schnell wie möglich auf −40°C eingefroren.

Die Bedingungen für die Gefriertrocknung sind für die einzelnen Größen der Injektionsflaschen unterschiedlich. Es gelten beispielsweise die folgenden Werte :

Dauer der Haupttrocknung bei einer Plattentemperatur von +15°C und 0,6 mbar :

| | |
|---|---|
| ca. 8-10 Stunden | für Gefäße mit 200 mg Ifosfamid + 200 mg Mesna |
| ca. 12-15 Stunden | für Gefäße mit 500 mg Ifosfamid + 500 mg Mesna |
| ca. 13-16 Stunden | für Gefäße mit 1 g Ifosfamid + 1 g Mesna |
| ca. 25-32 Stunden | für Gefäße mit 2 g Ifosfamid + 2 g Mesna |
| ca. 44-50 Stunden | für Gefäße mit 5 g Ifosfamid + 5 g Mesna |

Dauer der Nachtrocknung ca. 3-4 Stunden unter Vakuum von $5 \times 10^{-4}$ mbar, bei einer Plattentemperatur von 25°C. Die Restfeuchte (nach K. Fischer) soll unter 0,5% liegen. Nach Beendigung der Gefriertrocknung werden die Injektionsflaschen verschlossen. Zur Sicherung der Gummistopfen werden Bördelkappen aufgesetzt und anrolliert. Die fertigen Injektionsflaschen werden auf mechanische Defekte (Sprünge, fehlerhafter Verschluß etc.) kontrolliert.

## Patentansprüche

1. Lyophilisiertes Präparat, bestehend aus Ifosfamid, 0,05-1,0 Gewichtsteilen Mesna und 0,1 bis 17 Gewichtsteilen Hexit, Mesna und Hexit jeweils bezogen auf einen Gewichtsteil Ifosfamid sowie gegebenenfalls

anderen üblichen pharmazeutischen Hilfsstoffen.

2. Lyophilisiertes Präparat nach Anspruch 1, dadurch gekennzeichnet, daß es als Hexit Mannit enthält.

3. Verfahren zur Herstellung eines Ifosfamid-Mesna-Lyophilisates, dadurch gekennzeichnet, daß man unter einem inerten Gas eine wässrige oder wässrig-ethanolische Lösung, die 1 bis 13 Gewichtsprozent Ifosfamid enthält sowie 0,05-13 Gewichtsteile Mesna, 0,1 bis 17 Gewichtsteile Hexit (Mesna und Hexit jeweils bezogen auf einen Gewichtsteil Ifosfamid) und gegebenenfalls 0 bis 16,9 Gewichtsteile (bezogen auf 1 Gewichtsteil Ifosfamid) weitere pharmazeutische Hilfsstoffe, zwischen −70°C und 0°C einfriert, und dem so erhaltenen Produkt im gefrorenen Zustand das Wasser entzieht.

4. Verfahren nach Anspruch 3, dadurch gekennzeichnet, daß zuerst das nicht adsorptiv gebundene Wasser bei einer Temperatur zwischen −30°C und +40°C und einem Druck zwischen $10^{-3}$ bis 10 mbar und anschließend adsorptiv gebundenes Wasser bei einer Temperatur zwischen 0°C und 40°C und einem Druck zwischen $10^{-4}$ bis $10^{-1}$ mbar entfernt wird.

5. Verfahren nach einem oder mehreren der vorangegangenen Ansprüche, dadurch gekennzeichnet, daß als Hexit Mannit verwendet wird.

6. Ifosfamid-Mesna-Lyophilisat, erhalten nach einem oder mehreren der vorangegangenen Ansprüche.

## Claims

1. A lyophilized preparation consisting of ifosfamide, 0.05 to 1.0 parts by weight mesna and 0.1 to 17 parts by weight hexitol, the mesna and hexitol each being based on 1 part by weight ifosfamide, and optionally other typical pharmaceutical auxiliaries.

2. A lyophilized preparation as claimed in claim 1, characterized in that it contains mannitol as the hexitol.

3. A process for the production of an ifosfamide-mesna lyophilizate, characterized in that an aqueous or aqueous-alcoholic solution containing 1 to 13 parts by weight ifosfamide and 0.05 to 13 parts by weight mesna, 0.1 to 17 parts by weight hexitol (mesna and hexitol based on 1 part by weight ifosfamide) and optionally 0 to 16.9 parts by weight (based on 1 part by weight ifosfamide) other pharmaceutical auxiliaries are frozen between −70 and 0°C in an inert gas atmosphere and the water present in the product obtained is removed in the frozen state.

4. Process according to Claim 3, characterized in that the not adsorptively bound water is first removed at a temperature between −30°C and +40°C and a pressure between $10^{-3}$ to 10 mbar and that subsequently, adsorptively bound water is removed at a temperature between 0°C and 40°C and a pressure of between $10^{-4}$ and $10^{-1}$ mbar.

5. Process according to one or several of the preceding Claims, characterized in that the hexitol used is mannitol.

6. Ifosfamide-mesna lyophilisate obtained according to one or several of the preceding claims.

## Revendications

1. Préparation lyophilisée, se composant d'ifosfamide et, pour 1 partie en poids d'ifosfamide, de 0,05 à 1,0 partie en poids de mesna (2-mercaptoéthanesulfonate de sodium) et de 0,1 à 17 parties en poids d'hexitol, ainsi le cas échéant que d'autres adjuvants pharmaceutiques usuels.

2. Préparation lyophilisée selon la revendication 1, caractérisée en ce qu'elle contient, en tant qu'hexitol, du mannitol.

3. Procédé de préparation d'une composition lyophilisée à base d'ifosfamide et de mesna, caractérisé en ce que sous atmosphère d'un gaz inerte, on congèle entre −70°C et 0°C une solution aqueuse ou hydroéthanolique qui contient de 1 à 13% en poids d'ifosfamide et, par rapport à 1 partie en poids d'ifosfamide, de 0,05 à 13 parties en poids de mesna et de 0,1 à 17 parties en poids d'hexitol, ainsi le cas échéant que de 0 à 16,9 parties en poids (par rapport à 1 partie en poids d'ifosfamide) d'autre adjuvants pharmaceutiques, et on extrait l'eau à l'état gelé du produit ainsi obtenu.

4. Procédé selon la revendication 3, caractérisé en ce qu'on élimine d'abord l'eau non fixée par adsorption à une température comprise entre −30°C et +40°C et sous une pression comprise entre $10^{-3}$ et 10 mbars, puis on élimine l'eau fixée par adsorption à une température comprise entre 0°C et 40°C et sous une pression comprise entre $10^{-4}$ et $10^{-1}$ mbars.

5. Procédé selon l'une quelconque des revendications précédents caractérisé en ce qu'on utilise du mannitol en tant qu'hexitol.

6. Composition lyophilisée à base d'ifosfamide et de mesna, obtenue selon l'une quelconque des reven-

dications précédentes.